# EUROPEAN PATENT APPLICATION

(11) **EP 4 555 997 A1**
(43) Date of publication of application: **21.05.2025**
(21) Application number: 24213325.4
(22) Date of filing: 15.11.2024
(51) Int. Cl.: A61K 9/00, A61K 9/14, A61K 9/16, A61K 47/40, A61K 47/69, A61K 31/37, C08B 37/16

(54) **WATER-SOLUBLE COMPOSITION COMPRISING UROLITHIN**

(30) Priority: 15.11.2023 EP 23210042
(71) Applicant: Science4Beauty Sp. z o.o., 03-735 Warsaw (PL)
(72) Inventor: Dabrowa, Kajetan, 01-229 Warsaw (PL); Janczewska, Magdalena, 02-934 Warsaw (PL); Stefanek, Agata, 03-735 Warsaw (PL); Mazurkiewicz-Pisarek, Anna, 02-991 Warsaw (PL); Mazurkiewicz, Alina, 02-797 Warsaw (PL); Ciach, Tomasz, 02-765 Warsaw (PL)
(74) Representative: Kondrat, Mariusz

(57) **Abstract**

The invention relates to a water-soluble pharmaceutical or cosmetic composition comprising at least one urolithin substituted with a functionalized cyclodextrin **(F-CD)** and at least one cosmetically acceptable excipient, characterized in that weight ratio of urolithin to cyclodextrin **F-CD** is 0.5-14.0%, wherein cyclodextrin is **2-hydroxypropyl-β-cyclodextrin (HP-β-CD,** CAS no.: 128446-35-5), 2-hydroxypropyl γ-cyclodextrin (**HP-γ-CD**, CAS no.: 128446-34-4) or a combination thereof, and the urolithin is a compound of formula 1: 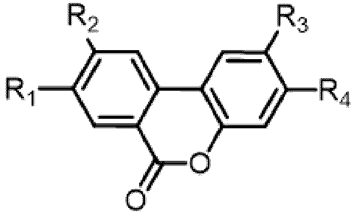 wherein: R₁ and R₄ are OH, and R₂ and R₃ are H; or R₄ is OH, and R₁, R₂ and R₃ are H; or R₁, R₂ and R₄ are OH, and R₃ is H; or R₁-R₄ is OH. The invention further relates to a method of making a composition according to the invention.

## Description

The object of the invention is a water-soluble pharmaceutical or cosmetic composition comprising urolithin and a method of manufacturing it.

Urolithins are hydroxy-6H-dibenzo[b,d]pyran-6-one derivatives and are formed by the transformation of ellagic acid and its derivatives such as ellagitannins by human intestinal colonic microflora (Larrosa M, González-Sarrías A, García-Conesa MT, Tomás-Barberán FA, Espín JC. Urolithins, Ellagic Acid-Derived Metabolites Produced by Human Colonic Microflora, Exhibit Estrogenic and Antiestrogenic Activities. J. Agric. Food Chem. 2006, 54, 5, 1611-1620).

They are found in the urine in the form of glucuronides after absorption of foods containing ellagitannins, such as pomegranate. During intestinal metabolism by bacteria, ellagitannins and punicalagins are converted to urolithins.

Within the meaning of the invention, urolithins include:
- Urolithin A **(UroA)** - 3,8-dihydroxy-6H-benzo[c]chromen-6-one (CAS No 1143-70-0);
- Urolithin B **(UroB)** - 3-hydroxy-6H-benzo[c]chromen-6-one (CAS No 1139-83-9);
- Urolithin C **(UroC)** - 3,8,9-Trihydroxy-6H-benzo[c]chromen-6-one (CAS No. 165393-06-6);
- Urofithin D **(UroD)** - 3,4,8,9-Tetaihydroxy-6H-dibenzo[b,d]pyran-6-one (CAS No. 131086-98-1).

The urofitins exhibit broad and diverse biological activities *in vitro* and *in vivo.* With that said, each of the A-D urolithins shows activity depending on the substitution site of the phenolic group in the aromatic rings (as indicated for example in the work: Kang I, Kim Y, Tomás-Barberán FA, Espín JC, Chung S. Urolithin A, C, and D, but not iso-urolithin A and urolithin B, attenuate triglyceride accumulation in human cultures of adipocytes and hepatocytes. Mol. Nutr. Food Res. 2016, 60: 1129-1138). Furthermore, these compounds exhibit anti-inflammatory and antioxidant properties *in vitro* and *in vivo,* with their antioxidant activity decreasing in a series: UroD > UroC > UroA > Uro B.

Due to their properties, urofitins are used in therapeutic applications. For example, application description EP3592347A1 discloses a method of treating or preventing a skin condition comprising topical administration of an effective amount of urolithin or its salt to a patient in need thereof. Wherein, said skin condition is selected from the group comprising melasma, chloasma, pregnancy mask, hyperpigmentation, skin aging, liver spots, lentigines, dermatitis, skin irritation, skin infections, warts, psoriasis, skin protection from environmental or therapy-induced damage, melanosis, dermatitis, linea nigra, Addison's and Cushing's syndrome. Wherein, melasma is advantageously selected from the group consisting of stress-related melasma, pregnancy-related melasma, hypothyroidism-associated melasma, melasma associated with administration of an active ingredient, melasma associated with exposure to sunlight or UV light, and melasma associated with exposure to a chemical agent..

In contrast, the major disadvantage of urolithins is their very low bioavailability, mainly due to their very low solubility in water and aqueous solutions *(*Sharma M, Li L, Celver J, Killian C, Kovoor A, Seeram NP. Effects of Fruit Ellagitannin Extracts, Ellagic Acid, and Their Colonic Metabolite, Urolithin A, on Wnt Signaling. J. Agric. Food Chem. 2010, 58, 7, 3965-3969). For example, the HPLC-determined solubility of **UroA** in water at 30° C is less than 0.01 g/L (JP6787633B2). For **UroB-D** urolithins, the theoretically determined solubilities are also very low and are 0.02 g/L, 0.20 g/L and 0.54 g/L, respectively (software: ChemAxon Solubility Predictor, method: (a) Hou, T. J.; Xia, K.; Zhang, W.; Xu, X. J. ADME Evaluation in Drug Discovery. 4th Prediction of Aqueous Solubility Based on Atom Contribuition Approach. J. Chem. Inf. Comput. Sci. 2004, 44, 266-275. (b) Shoghi, E.; Fuguet, E.; Bosch, E.; Rafols, C. Solubility-pH profiles of some acidic, basic and amphoteric drugs European Journal of Pharmaceutical Sciences 2013, 48, 291-300).

Due to the above, there is a great need to improve the water solubility of urolithins. In this context, international application description WO2022063846 A1 indicates that the insolubility of urolithin in water poses a major challenge in its use as an active ingredient, which is related to obtaining urolithin as a suspension when the powder is dissolved in water. The insolubility of urolithins promotes almost immediate deposition of urolithin at the bottom of the reconstitution container. In this context, the said prior art application includes various ways of stabilising urolithin A in an aqueous matrix so that when the powder containing urolithin A is reconstituted and the urolithin remains in suspension. Whereby, the method discloses therein comprises an use of xanthan gum in the stabilisation of urolithin.

The patent literature also indicates the usefulness of cyclodextrins in improving the water solubility of sparingly soluble active substances. For example, the international patent application WO2010110328 A1 discloses the use of β- or γ-cyclodextrin in improving the water solubility of hard-to-solubilise flavonoids is known.

Japanese application JP2008271839 A discloses a method of increasing the solubility of a flavonoid by using a combination of β-cyclodextrin and α-glucosylhesperidin. Whereby, the favourable quantitative ratio of the difficult-to-solubilise flavonoid to β-cyclodextrin and α-glucosylhesperidin was 1 mol to 1.5-5.0 mol and 0.1-1.0 mol.

On the other hand, Japanese patent JP6787633B2 discloses a method of increasing the solubility of urolithin A in water by using unsubstituted (native) α-, β- and γ-cyclodextrin (CD). However, the method disclosed therein resulted in an unsatisfactory increase in the water solubility of urolithin (in the range of 1.2-14.8). Furthermore, it required the use of very large amounts of cyclodextrins - i.e. 364-3196 parts cyclodextrin to urolitin A are required; in other words, the mass percentage of **UroA** urolitin in the UroA/cyclodextrin mixture is only 0.03-0.27%.

Accordingly, the currently provided methods for increasing the solubility of urolithins in water are not sufficiently practical and efficient. Therefore, it is an object of the invention to provide a method for increasing the solubility of urolithin in water and compositions comprising urolithin.

The essence of the invention is a water-soluble pharmaceutical or cosmetic composition comprising at least one urolithin substituted with a functionalized cyclodextrin **(F-CD)** and at least one cosmetically acceptable excipient, characterized in that weight ratio of urolithin to cyclodextrin **F-CD** is 0.5-14.0%, wherein cyclodextrin is **2-hydroxypropyl-β-cyclodextrin (HP-β-CD,** CAS no.: 128446-35-5), 2-hydroxypropyl γ-cyclodextrin **(HP-γ-CD,** CAS no.: 128446-34-4) or a combination thereof, and the urolithin is a compound of formula I: 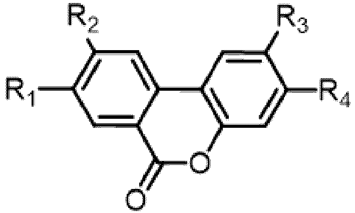 wherein: R₁ and R₄ are OH, and R₂ and R₃ are H; or R₄ is OH and R₁, R₂ and R₃ are H; or R₁, R₂ and R₄ are OH and R₃ is H; or R₁-R₄ is OH.Another essence of the invention is a method of manufacturing a composition according to the invention, characterised in that it comprises the following steps:
1) Preparation of a mixture of at least one urolithin and cyclodextrin **F-CD** in water **M1**, an organic solvent **M2,** a mixture of organic solvents **M3** or in a mixture of water and organic solvents **M4** with or without the addition of inorganic salts **D1** or buffering compounds **D2;**
2) Mixing of the mixture prepared in step 1 at a temperature of 4-60 °C, preferably at 15-25 °C, for a period of 1-96 hours, preferably 12 hours;
3) Filtering insoluble components of the prepared mixture through a filter with a pore size in the range of 0.22-0.80 *µ*m, preferably 0.22-0.45 *µ*m;
4) Evaporation of volatile components of the mixture under reduced pressure at 15-50 °C, preferably 15-30 °C;
5) Drying and micronisation of the resulting multi-component precipitate **P1.**

Advantageously, the method according to the invention comprises an additional processing of the precipitate **P1** obtained in step 5, wherein additional processing comprises following steps:
a) Suspension of precipitate **P1** in water with a pH in the range 3.5-8.5, preferably with a pH in the range 6.5-7.5;
b) stirring the mixture prepared in step a) in the dark at a temperature of 4-30 °C, preferably 10-25 °C, for a period of 1-96 hours, preferably 2-12 hours;
c) Filtering the insoluble components of the prepared mixture through a filter with a pore size in the range of 0.22-0.80 *µ*m, preferably 0.22-0.45 *µ*m;

Advantageously, the organic solvent **M2** used in step 1) of the method according to the invention is selected from the group comprising methanol (MeOH), ethanol (EtOH), tetrahydrofuran (THF), and 2-methyltetrahydrofuran (2-THF), advantageously MeOH and THF.

Advantageously the solvent mixture **M3** used in step 1) of the method according to the invention is a mixture of two organic solvents **M2** in the range of mutual volume ratio 1:9 - 9:1 (v/v), advantageously the solvent mixture **M3** used in step 1) of the method is a mixture of MeOH and THF in a volume ratio of 4:1 (v/v).

Advantageously the mixture of water and organic solvent **M4** used in step 1) of the method according to the invention is a mixture of water and a single organic solvent **M2** or a mixture of organic solvents **M3** in the range of mutual volume ratio 1:9 - 9:1 (v/v), advantageously a mixture of water and THF in a volume ratio of 1:3 - 1:9 (v/v), a mixture of water and MeOH in a volume ratio of 1:3 - 1:9 (v/v) or a mixture of water, MeOH and THF in a volume ratio of 1:1:1 - 1:1.8:7.2 (v/v/v).

Advantageously the inorganic salt **D1** used in step 1) according to the invention is sodium chloride (NaCl), sodium sulphate (Na₂SO₄), sodium bicarbonate (NaHCOs), potassium bicarbonate (KHCOs), sodium dihydrogen phosphate (NaH₂PO₄), potassium dihydrogen phosphate (KH₂PO₄), or a combination thereof, advantageously the inorganic salt used in step 1) of the method according to the invention is a combination of NaCl and Na₂SO₄.

Advantageously, the buffering compound **D1** used in step 1) of the method according to the invention is EDTA, Na₂CO₃ /NaHCO₃, K₂CO₃ /KHCO₃, NaH₂PO₄ /Na₂HPO₄, KH₂PO₄ /K₂HPO₄ or a combination thereof.

The invention provides the following benefits:
- The method according to the invention enables obtaining compositions comprising highly water-soluble urolithin, which enables its use in the cosmetic and/or pharmaceutical industry;
- Increasing the solubility of urolithin using cyclodextrins requires the use of much less cyclodextrin than in known methods, essentially reducing the cost of the technology;

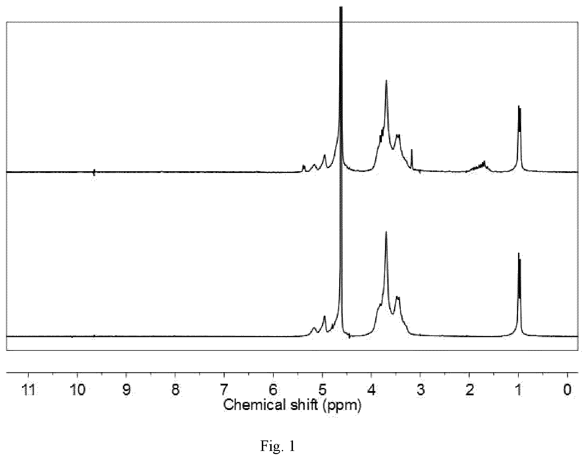

The invention is illustrated in working examples in the figure, wherein fig. 1 shows the¹ H NMR spectrum of an inclusion type complex between urolithin A **(UroA)** and hydroxypropyl-γ-cyclodextrin **(HP-γ-CD)** in deuterated water.

### Example 1.

Composition comprising UroA and HP-β-CD in concentration variants of 0.5%, 7.0%, and 14.0%.

**Table 1. Quantitative and qualitative composition of the compositions in variants P1, P2 and P3.**

| Variant UroA concentration (% m/m) | | Concentration of HP-P-CD (% m/m) |
|---|---|---|
| **P1** | 0.5% | 99.5% |
| **P2** | 7.0% | 93.0% |
| **P3** | 14.0% | 86.0% |

### Preparation of the composition:

1. An appropriate amount of HP-β-CD was added to a 100 mL flask.
2. HP-β-CD was dissolved in 90 mL of distilled water while stirring (500 rpm, temperature 25°C).
3. UroA was added in portions to the clear solution, continuing to stir for 2 hours at 25°C.
4. The mixture was freeze-dried to obtain a powder in variants P1, P2 and P3.

### Example 2.

Composition comprising UroB and HP-γ-CD in concentration variants of 0.5%, 7.0%, and 14.0%.

**Table 2. Quantitative and qualitative composition of the compositions in variants P4, P5 and P6.**

| Variant | UroB concentration (% m/m) | Concentration of HP-γ-CD (% m/m) |
|---|---|---|
| **P4** | 0.5% | 99.5% |
| **P5** | 7.0% | 93.0% |
| **P6** | 14.0% | 86.0% |

### Preparation of the composition:

1. An appropriate amount of HP-γ-CD was added to a 100 mL flask.
2. HP-γ-CD was dissolved in 90 mL of distilled water while stirring (500 rpm, temperature 25°C).
3. UroB was added in portions to the clear solution, continuing stirring for 2 hours at 25°C.
4. The mixture was freeze-dried to obtain powder variants P4, P5 and P6.

### Example 3.

Composition comprising UroC and HP-β-CD in concentration variants of 0.5%, 7.0%, and 14.0%.

**Table 3. Quantitative and qualitative composition of the compositions in variants P7, P8 and P9.**

| Variant | UroC concentration (% m/m) | Concentration of HP-P-CD (% m/m) |
|---|---|---|
| **P7** | 0.5% | 99.5% |
| **P8** | 7.0% | 93.0% |
| **P9** | 14.0% | 86.0% |

### Preparation of the composition:

1. An appropriate amount of HP-β-CD was added to a 100 mL flask.
2. HP-β-CD was dissolved in 90 mL of distilled water while stirring (500 rpm, temperature 25°C).
3. UroC was added in portions to the clear solution, continuing to stir for 2 hours at 25°C.
4. The mixture was freeze-dried to obtain powder variants P7, P8 and P9.

### Example 4.

Composition comprising UroD and HP-γ-CD in concentration variants of 0.5%, 7.0%, and 14.0%.

**Table 4. Quantitative and qualitative composition of the compositions in variants P10, P11 and P12.**

| Variant | UroD concentration (% m/m) | Concentration of HP-γ-CD (% m/m) |
|---|---|---|
| **P10** | 0.5% | 99.5% |
| **P11** | 7.0% | 93.0% |
| **P12** | 14.0% | 86.0% |

### Preparation of the composition:

1. An appropriate amount of HP-γ-CD was added to a 100 mL flask.
2. HP-γ-CD was dissolved in 90 mL of distilled water while stirring (500 rpm, temperature 25°C).
3. UroD was added in portions to the clear solution, continuing to stir for 2 hours at 25°C.
4. The mixture was freeze-dried to obtain powder variants P10, P11 and P12.

### Example 5.

Composition comprising UroA and HP-γ-CD in concentration variants of 0.5%, 7.0%, and 14.0%.

**Table 5. Quantitative and qualitative composition of the compositions in variants P13, P14 and P15.**

| Variant | UroA concentration (% m/m) | Concentration of HP-γ-CD (% m/m) |
|---|---|---|
| **P13** | 0.5% | 99.5% |
| **P14** | 7.0% | 93.0% |
| **P15** | 14.0% | 86.0% |

### Preparation of the composition:

1. An appropriate amount of HP-γ-CD was added to a 100 mL flask.
2. HP-γ-CD was dissolved in 90 mL of distilled water while stirring (500 rpm, temperature 25°C).
3. UroA was added in portions to the clear solution, continuing stirring for 2 hours at 25°C.
4. The mixture was freeze-dried to obtain powder variants P13, P14 and P15.

### Example 6.

Composition comprising UroB and HP-β-CD in concentration variants of 0.5%, 7.0%, and 14.0%.

**Table 6. Quantitative and qualitative composition of the compositions in variants P16, P17 and P18.**

| Variant | UroB concentration (% m/m) | Concentration of HP-P-CD (% m/m) |
|---|---|---|
| **P16** | 0.5% | 99.5% |
| **P17** | 7.0% | 93.0% |
| **P18** | 14.0% | 86.0% |

### Preparation of the composition:

1. An appropriate amount of HP-β-CD was added to a 100 mL flask.
2. HP-β-CD was dissolved in 90 mL of distilled water while stirring (500 rpm, temperature 25°C).
3. UroB was added in portions to the clear solution, continuing stirring for 2 hours at 25°C.
4. The mixture was freeze-dried to obtain powder variants P16, P17 and P18.

### Example 7.

Mixture of UroA and UroB with HP-β-CD in concentration variants of 0.25% UroA + 0.25% UroB (total 0.5%), 3.5% UroA + 3.5% UroB (total 7.0%), and 7.0% UroA + 7.0% UroB (total 14.0%).

**Table 7. Quantitative and qualitative composition of the compositions in variants P19, P20 and P21.**

| Variant | 1 UroA concentration (% m/m) | 1 UroB concentration (% m/m) | Concentration of HP-P-CD (% m/m) |
|---|---|---|---|
| **P19** | 0.25% | 0.25% | 99.5% |
| **P20** | 3.5% | 3.5% | 93.0% |
| **P21** | 7.0% | 7.0% | 86.0% |

### Preparation of the composition:

1. An appropriate amount of HP-β-CD was added to a 100 mL flask.
2. HP-β-CD was dissolved in 90 mL of distilled water while stirring (500 rpm, temperature 25°C).
3. To the clear solution, UroA and UroB were added in portions in appropriate proportions, continuing to stir for 2 hours at 25°C.
4. The mixture was freeze-dried to obtain powder variants P19, P20 and P21.

### Example 8.

Mixture of UroC and UroD with HP-γ-CD in concentration variants of 0.25% UroC + 0.25% UroD (total 0.5%), 3.5% UroC + 3.5% UroD (total 7.0%), and 7.0% UroC + 7.0% UroD (total 14.0%).

**Table 8. Quantitative and qualitative composition of the compositions in variants P22, P23 and P24.**

| Variant | UroC concentration (% m/m) | UroD concentration (% m/m) | Concentration of HP-γ-CD (% m/m) |
|---|---|---|---|
| **P22** | 0.25% | 0.25% | 99.5% |
| **P23** | 3.5% | 3.5% | 93.0% |
| **P24** | 7.0% | 7.0% | 86.0% |

### Preparation of the composition:

1. An appropriate amount of HP-γ-CD was added to a 100 mL flask.
2. HP-γ-CD was dissolved in 90 mL of distilled water while stirring (500 rpm, temperature 25°C).
3. To the clear solution, UroC and UroD were added in portions in appropriate proportions, continuing to stir for 2 hours at 25°C.
4. The mixture was freeze-dried to obtain powder variants P22, P23 and P24.

### Example 9.

In this working example, a method of manufacturing a composition according to the invention is shown using the minimum parameter values indicated in the method claims, and using different solvents, salts and buffers.
1. Preparation of the mixture:
   To a 250 mL round-bottom flask containing distilled water (**M1**, 100 mL), urolithin A (UroA, 0.50 g, 2.2 mmol) and 2-hydroxypropyl-β-cyclodextrin (HP-β-CD, 5.00 g, ~3.2 mmol) were added. The inorganic salt NaCl (**D1**, 0.10 g) and the buffering compound EDTA **(D2,** 0.01 g) were then added.
2. Mixing:
   The contents of the flask were stirred magnetically (500 rpm) at 4°C for 96 hours in the dark.
3. Filtration:
   The mixture was filtered through a 0.80 µm pore size filter.
4. Evaporation:
   The volatile components of the mixture were evaporated under reduced pressure at 15°C.
5. Drying and micronisation:
   The resulting precipitate (P25) was dried in a vacuum dryer at 15°C for 24 hours and micronised to obtain P25 as a fine powder.

### Example 10.

In this working example, a method manufacturing a composition according to the invention is shown using maximum parameter values and different solvents and salts.

Preparation of the composition:
1. Preparation of the mixture:
   To a 250 mL round-bottom flask containing a mixture of tetrahydrofuran and methanol (M3, 90 mL, ratio 9:1 v/v), urolithin B (UroB, 0.50 g, 2.2 mmol) was added while stirring (500 rpm). Once UroB was completely dissolved, 2-hydroxypropyl-γ-cyclodextrin (HP-γ-CD, 5.00 g, ~3.2 mmol) was added. The inorganic salt Na₂SO₄ (**D1**, 1.00 g) and the buffer NaH₂ PO4/Na₂ HPO₄ (D2, 1.00 g) were then added.
2. Mixing:
   The contents of the flask were stirred magnetically (500 rpm) at 60°C for 1 hour.
3. Filtration:
   The mixture was filtered through a 0.22 µm pore size filter.
4. Evaporation:
   The volatile components of the mixture were evaporated under reduced pressure at 50°C.
5. Drying and micronisation:
   The resulting precipitate (P26) was dried in a vacuum dryer at 50°C for 12 hours and micronised to obtain P26 as a fine powder.

### Example 11.

In this working example, a method of manufacturing a composition is shown using different proportions of **M4** solvents and inorganic salts.

### Preparation of the composition with M4 in a ratio of 1:9 (water:THF):

1. Preparation of the mixture:
   Distilled water (M4, 10 mL) and tetrahydrofuran (90 mL) were added to a 250 mL flask to form a mixture with a volume ratio of 1:9 (v/v). Urofithin C (UroC, 0.50 g, 2.2 mmol) and HP-β-CD (5.00 g, ~3.2 mmol) were then added. The inorganic salt NaCl (D1, 0.50 g) and Na2SO4 (D1, 0.50 g) were added.
2. Mixing:
   The contents of the flask were stirred magnetically (500 rpm) at 25°C for 12 hours.
3. Filtration and evaporation:
   This step was performed as in the previous examples, yielding a precipitate (P27).

### Preparation of a composition with M4 in a 9:1 ratio (water:THF):

1. Preparation of the mixture:
   Distilled water **(M4,** 90 mL) and tetrahydrofuran (10 mL) were added to a 250 mL flask to form a mixture with a volume ratio of 9:1 (v/v). Urofithin D (UroD, 0.50 g, 2.2 mmol) and HP-γ-CD (5.00 g, ~3.2 mmol) were then added. KH₂PO₄/K₂HPO₄ buffer **(D2,** 1.00 g) was added.
2. Mixing:
   The contents of the flask were stirred magnetically (500 rpm) at 25°C for 12 hours.
3. Filtration and evaporation:
   This step was performed as in the previous examples, yielding a precipitate (P28).

### Example 12.

In this working example, a method of manufacturing a composition is shown using different organic solvents **M2** and **M3** and buffer mixtures.

### Preparation of a composition with M2 (methanol):

1. Preparation of the mixture:
   Methanol **(M2,** 100 mL) was added to a 250 mL round-bottom flask. Then urolithin A (UroA, 0.50 g, 2.2 mmol) was added while stirring (500 rpm). After dissolution of UroA, HP-β-CD (5.00 g, ~3.2 mmol) was added. Na₂ COs /NaHCO₃ buffer **(D2,** 1.00 g) was added.
2. Mixing:
   The contents of the flask were stirred magnetically at 25°C for 12 hours.
3. Filtration and evaporation:
   Proceeded as before, yielding a sediment (P29).

### Preparation of the composition with M3 (mixture of MeOH and THF in a ratio of 4:1 v/v):

1. Preparation of the mixture:
   Methanol (80 mL) and tetrahydrofuran (20 mL) were added to a 250 mL round-bottom flask to form an **M3** mixture with a 4:1 (v/v) volume ratio. Urofithin B (UroB, 0.50 g, 2.2 mmol) was then added. Once UroB was completely dissolved, HP-γ-CD (5.00 g, ~3.2 mmol) was added. EDTA buffering compound (D2, 0.01 g) was added.
2. Mixing:
   The contents of the flask were stirred magnetically at 25°C for 12 hours.
3. Filtration and evaporation:
   Proceeded as before, obtaining sediment (P30).

### Example 13.

In this working example, a method of manufacturing a composition is shown using different salts and buffers and different mixing temperatures and times.

### Preparation of the composition with KH₂PO₄/K₂HPO₄ buffer and maximum mixing time:

1. Preparation of the mixture:
   To a 250 mL flask containing distilled water (100 mL) was added urolithin C (UroC, 0.50 g, 2.2 mmol) and HP-β-CD (5.00 g, ~3.2 mmol). Buffer KH₂ PO /K₄₂ HPO₄ **(D2, 1.00** g) was added, setting the pH to 7.0.
2. Mixing:
   The contents of the flask were stirred magnetically at 25°C for 96 hours.
3. Filtration and evaporation:
   Proceeded as in the previous examples, obtaining a precipitate (P31).

### Preparation of the composition with NaHCO₃ salt and minimum mixing time:

1. Preparation of the mixture:
   To a 250 mL flask containing distilled water (100 mL) was added urolithin D (UroD, 0.50 g, 2.2 mmol) and HP-γ-CD (5.00 g, ~3.2 mmol). NaHCO₃ salt (**D1**, 0.10 g) was added.
2. Mixing:
   The contents of the flask were stirred magnetically at 25°C for 1 hour.
3. Filtration and evaporation:
   Proceeded as before, obtaining a precipitate (P32).

The above examples 9-13 illustrate the use of the extreme values of the parameters indicated in the claims of the method of manufacturing of the composition according to the invention, including the use of different solvents (**M1**, **M2, M3, M4),** inorganic salts (**D1**) and buffering compounds **(D2)** in specific proportions and process conditions.

### Example 14.

*Mixture of UroA, UroB, UroC and UroD with HP-β-CD.*

**Table 9. Quantitative and qualitative composition of the compositions in variants P33-P35.**

| **Variant** | **UroA concentration (% m/m)** | **UroB concentration (% m/m)** | **UroC concentration (% m/m)** | **UroD concentration (%m/m)** | **Concentration of HP-β-CD (% m/m)** |
|---|---|---|---|---|---|
| P33 | 0.125% | 0.125% | 0.125% | 0.125% | 99.5% |
| P34 | 1.75% | 1.75% | 1.75% | 1.75% | 93.0% |
| P35 | 3.5% | 3.5% | 3.5% | 3.5% | 86.0% |

### Preparation of the composition:

1. Distilled water (100 mL) was added to a 250 mL flask.
2. HP-β-CD (5.00 g, ~3.2 mmol) was dissolved in water while stirring (500 rpm, temperature 25°C).
3. Portions of UroA, UroB, UroC and UroD were added to the HP-β-CD solution in equal proportions (total 0.50 g, -2.2 mmol).
4. The mixture was stirred magnetically at 25°C for 2 hours.
5. The clear solution was freeze-dried to obtain the composition as a fine powder in variants P33, P34 and P35.

### Example 15.

*Mixture of UroB and UroD with HP-γ-CD and HP-β-CD.*

**Table 10. Quantitative and qualitative composition of the compositions in variants P36, P37 and P38.**

| **Variant** | **UroB concentration (% m/m)** | **UroD concentration (% m/m)** | **Concentration of HP-γ- CD (% m/m)** | **Concentration of HP-β-CD (% m/m)** |
|---|---|---|---|---|
| P36 | 0.25% | 0.25% | 49.75% | 49.75% |
| P37 | 3.5% | 3.5% | 46.5% | 46.5% |
| P38 | 7.0% | 7.0% | 43.0% | 43.0% |

### Preparation of the composition:

1. Distilled water (100 mL) was added to a 250 mL flask.
2. HP-γ-CD (2.50 g, -1.6 mmol) and HP-β-CD (2.50 g, ~1.6 mmol) were dissolved in water while stirring (500 rpm, temperature 25°C).
3. Portions of UroB (0.25 g, ~1.1 mmol) and UroD (0.25 g, -1.1 mmol) were added to the cyclodextrin solution.
4. The mixture was stirred magnetically at 25°C for 2 hours.
5. The clear solution was lyophilised to obtain the composition as a fine powder in variants P36, P37 and P38.

## Claims

1. A water-soluble pharmaceutical or cosmetic composition comprising at least one urolithin, substituted with a functionalized cyclodextrin **(F-CD)** and at least one cosmetically acceptable excipient, **characterized in that** weight ratio of urolithin to cyclodextrin **F-CD** is 0.5-14.0%, wherein cyclodextrin is **2-hydroxypropyl-β-cyclodextrin (HP-β-CD,** CAS no.: 128446-35-5), 2-hydroxypropyl γ-cyclodextrin **(HP-γ-CD,** CAS no.: 128446-34-4) or a combination thereof, and the urolithin is a compound of formula I: 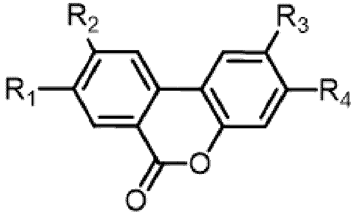 wherein: R₁ and R₄ are OH, and R₂ and R₃ are H; or R₄ is OH and R₁, R₂ and R₃ are H; or R₁, R₂ and R₄ are OH and R₃ is H; or R₁-R₄ is OH.

2. Water-soluble composition according to claim 1, **characterised in that** urolithin is urolithin A.

3. A method of manufacturing a composition as defined in claim 1 or 2, **characterised in that** it comprises the following steps:
a) Preparation of a mixture of at least one urolithine and cyclodextrin **(F-CD)** in water (**M1**), an organic solvent **(M2),** a mixture of organic solvents **(M3)** or in a mixture of water and organic solvents **(M4)** with or without the addition of inorganic salts (**D1**) or buffering compounds **(D2);**
b) Mixing of the mixture prepared in step 1 at a temperature of 4-60 °C, preferably 15-25 ° C, for a period of 1-96 hours, preferably 12 hours;
c) Filtering insoluble components of the prepared mixture through a filter with a pore size in the range of 0.22-0.80 *µ*m, preferably 0.22-0.45 *µ*m;
d) Evaporation of volatile components of the mixture under reduced pressure at 15-50 °C, preferably 15-30 °C;
e) Drying and micronisation of the resulting multicomponent precipitate (**P1**).

4. The method for producing the composition as defined in claim 3, **characterised in that** it comprises an additional processing of the precipitate (**P1**) obtained in step 5, wherein additional processing comprises following steps:
a) Suspension of the precipitate (**P1**) in water with a pH between 3.5 and 8.5, preferably with a pH between 6.5 and 7.5;
b) stirring the mixture prepared in step a) in the dark at a temperature of 4-30 °C, preferably 10-25 °C, for a period of 1-96 hours, preferably 2-12 hours;
c) Filtering the insoluble components of the prepared mixture through a filter with a pore size in the range of 0.22-0.80 *µ*m, preferably 0.22-0.45 *µ*m;

5. The method according to claim 3, **characterised in that** the organic solvent **(M2)** used in step a) is selected from the group comprising methanol (MeOH), ethanol (EtOH), tetrahydrofuran (THF), and 2-methyltetrahydrofuran (2-THF), preferably MeOH and THF.

6. The method according to claim 3, **characterised in that** the solvent mixture **(M3)** used in step a) is a mixture of two organic solvents **(M2)** in the range of mutual volume ratio 1:9 - 9:1 (v/v), preferably the solvent mixture **(M3)** used in step 1) is a mixture of MeOH and THF in a volume ratio of 4:1 (v/v).

7. The method according to claim 3, **characterised in that** the mixture of water and organic solvent **(M4)** used in step a) is a mixture of water and a single organic solvent **(M2)** or a mixture of organic solvents **(M3)** in the range of mutual volume ratio 1:9 - 9:1 (v/v), advantageously a mixture of water and THF in a volume ratio of 1:3 - 1:9 (v/v), a mixture of water and MeOH in a volume ratio of 1:3 - 1:9 (v/v) or a mixture of water, MeOH and THF in a volume ratio of 1:1:1 - 1:1.8:7.2 (v/v/v).

8. The method according to claim 3, **characterised in that** the inorganic salt **D1** used in step a) is sodium chloride (NaCl), sodium sulphate (Na₂SO₄), sodium bicarbonate (NaHCOs), potassium bicarbonate (KHCOs), sodium dihydrogen phosphate (NaH₂PO₄), potassium dihydrogen phosphate (KH₂PO₄), or a combination thereof, advantageously the inorganic salt used in step 1) is a combination of NaCl and Na₂SO₄.

9. The method according to claims 3 to 7, **characterised in that** the buffering compound **D1** used in step a) of the method according to the invention is EDTA, Na₂CO₃ /NaHCO₃, K₂ CO₃/KHCO₃, NaH₂PO₄ /Na₂HPO₄, KH₂PO₄ /K₂HPO₄ or a combination thereof.
